# EUROPÄISCHE PATENTSCHRIFT

(11) **EP 2 184 075 B1**
(45) Veröffentlichungstag und Bekanntmachung des Hinweises auf die Patenterteilung: **19.10.2011**
(21) Anmeldenummer: 09173786.6
(22) Anmeldetag: 22.10.2009
(51) Int. Cl.: A61L 2/03, A61L 2/18, A22B 7/00

(54) **Verfahren zur Reinigung eines Mittels zur Behandlung von Lebensmitteln**
Method for cleaning a food handling device
Procédé de nettoyage d'un produit de traitement d'aliments

(30) Priorität: 30.10.2008 DE 102008054032
(43) Veröffentlichungstag der Anmeldung: 12.05.2010
(73) Patentinhaber: Aquagroup Ag, 93055 Regensburg (DE)
(72) Erfinder: Czech, Manuel, 93093, Donaustauf (DE); Philipps, André, 93059, Regensburg (DE); Saefkow, Michael, 74189, Weinsberg (DE)
(74) Vertreter: Bublak, Wolfgang

(56) Entgegenhaltungen:
- EP-A1- 1 495 770
- WO-A2-02/058449
- DE-A1-102007 017 502
- US-A- 6 117 285
- US-A1- 2003 131 426

## Beschreibung

Die vorliegende Erfindung betrifft ein Verfahren zur Reinigung eines Mittels zur Behandlung von Lebensmitteln, mittels einer Vorrichtung, die Teil eines Schlacht und/oder Zerlegebetriebes ist, und wobei die Vorrichtung wenigstens einen Flüssigkeitszulauf sowie ein Mittel zur Beaufschlagung der Oberflächen des Mittels zur Behandlung von Lebensmitteln mit einer Reinigungsflüssigkeit umfasst.

Bei der Behandlung beziehungsweise Verarbeitung von Lebensmitteln sind hohe Ansprüche an die Reinigung der verwendeten Hilfsmittel zu stellen, um eine zufriedenstellende Hygiene und Qualität der Lebensmittel zu gewährleisten. Dies ist insbesondere bei der Verarbeitung von tierischen Produkten erforderlich, da hier aufgrund der von Natur aus bestehenden höheren Keimdichte Mängel bei der Hygiene schneller zu Kontaminationen und Gesundheitsrisiken führten können. Insbesondere im Schlachtbetrieb ist zu gewährleisten, dass die Mittel zur Behandlung der tierischen Produkte, wie zum Beispiel Messer, während des Schlachtvorganges ständig und zuverlässig gereinigt und dekontaminiert werden, um eine Übertragung von Erregern von einem Tier auf ein anderes Tier zu vermeiden. Hierzu wird im Stand der Technik im Schlachtbetrieb die sogenannte Zweimessertechnik angewandt: Hierbei öffnet der Metzger ein erstes Tier mit einem ersten Messer auf, welches er dann in ein Heißwasserbad steckt. Mit einem zweiten Messer wird ein zweites Tier geöffnet, und das Messer anschließend. in dasselbe Heißwasserbad gesteckt. Ein drittes Tier wird dann wiederum mit dem ersten Messer geöffnet. Durch das Wasserbad sollen Keime abgetötet und Verschmutzungen abgelöst werden und so eine Übertragung der Keime von einem Tier auf das nächste Tier unterbunden werden. Solche Wasserbäder werden als "Sterilbecken" bezeichnet. Bei der Verwendung solcher "Sterilbecken" ist eine Temperatur von 82°C gesetzlich vorgeschrieben. Durch das Becken soll zusätzlich ein konstanter Heißwasserstrom fließen; außerdem ist der Einbau einer elektrischen Heizspirale üblich, die das Wasser nachheizt.

Ein Gerät und Verfahren zur Sterilisation von Messern ist u.a. aus EP-A-1 495 770 und US 2003/0131426 bekannt.

Diese im Stand der Technik zugänglichen Vorrichtungen weisen den Nachteil auf, dass häufig die vorgeschriebene Temperatur von 82°C im Becken nicht eingehalten werden kann. Darüber hinaus reduzieren die Anwender häufig die Temperatur, da die Mittel zur Behandlung von Lebensmitteln sonst als zu heiß in der Hand empfunden werden. Unter diesen Voraussetzungen entwickelt sich im Heißwasserbad schnell eine keimhaltige Fleischbrühe, welche Keime von einem kranken Tier auf alle anderen gesunden Tiere übertragen kann. Daher sind solche Einrichtungen unbefriedigend, mangels einer Alternative aber immer noch im Einsatz.

Hiervon ausgehend ist es Aufgabe der vorliegenden Erfindung, ein verbessertes Verfahren zur Reinigung eines Mittels zur Behandlung von Lebensmitteln zur Verfügung zu stellen.

Gelöst wird diese Aufgabe mit den Merkmalen des Patentanspruches 1. Vorteilhafte Aus- und Weiterbildungen, welche einzeln oder in Kombination miteinander eingesetzt werden können, sind Gegenstand der Unteransprüche.

Das erfindungsgemäße Verfahren baut auf den Merkmalen des Oberbegriffes dadurch auf, dass über das Mittel zur Beaufschlagung der Oberflächen des Mittels zur Behandlung von Lebensmitteln mit einer Reinigungsflüssigkeit eine elektrochemisch behandelte Flüssigkeit einsprühbar ist.

Dadurch wird gegenüber dem Stand der Technik eine erhebliche Verbesserung erzielt. Dadurch, dass über das Mittel zur Beaufschlagung der Oberfläche mit einer Reinigungsflüssigkeit eine mittels Elektrolyse gewonnene Flüssigkeit einsprühbar ist, wird eine umfassende und zuverlässige Desinfektion des Mittels zur Behandlung von Lebensmitteln erzielt. Dabei erfolgt die Desinfektion primär nicht durch Wärmzufuhr sondern durch die Eigenschaften der elektrochemisch behandelten Reinigungsflüssigkeit. Mit jedem neu eingebrachten Mittel zur Behandlung von Lebensmitteln wird ein neuer Desinfektionsvorgang gestartet, dessen Desinfektionserfolg unabhängig von der Anzahl der zuvor getätigten Desinfektionsmaßnahmen ist. Ein Kumulationseffekt, wie er zum Beispiel bei dem oben genannten Sterilbecken auftritt, bei dem mit einer zunehmenden Zahl erfolgter Reinigungen die Qualität des Reinigungswassers im Sterilbecken und damit die Qualität der Desinfektionen sukzessiv abnimmt, wird durch das erfindungsgemäße Verfahren ausgeschlossen. Dies wird dadurch ermöglicht, dass das Mittel zur Behandlung von Lebensmitteln nicht in einem Bad, sondern durch Beaufschlagung mit der Reinigungsflüssigkeit gereinigt wird bzw. abgespritzt wird. Ein weiterer Vorzug der Erfindung gegenüber dem Stand der Technik besteht darin, dass die Temperatur des Mittels zur Behandlung von Lebensmitteln für den Anwender angenehmer ist, da nicht eine Erhitzung auf 82°C zwingend vorgenommen werden muss, um eine Desinfektion zu gewährleisten. Für den Anwender besteht somit auch keine Veranlassung, die Temperatur zu reduzieren, was den Desinfektionserfolg vermindern könnte.

Ebenso bewirkt das erfindungsgemäße Verfahren eine erhebliche Energieeinsparung, da nicht wie im Stand der Technik üblich ein großes Wasserbecken auf einer hohen Temperatur gehalten werden muss. Schließlich ist eine Überwachung der Temperatur in einem Sterilbecken im Stand der Technik schwierig; in einem solchen Becken ist aber die Temperatur entscheidend für den Desinfektionserfolg. Bei der erfindungsgemäßen Vorrichtung hingegen kann die Temperatur der Reinigungsflüssigkeit auf einfache und zuverlässige Weise ermittelt und gesteuert bzw. geregelt werden.

In einer bevorzugten Ausführungsform ist die Gesamtkonzentration der bei der Elektrolyse der Reinigungsflüssigkeit entstandenen Oxidantien kleiner als 500 ppm, bevorzugt kleiner als 200 ppm, besonders bevorzugt kleiner als 20 ppm, ganz besonders bevorzugt kleiner als 2 ppm, und insbesondre besonders bevorzugt kleiner 0,2 ppm ist. Durch diesen geringen Gehalt an verbleibenden Oxidantien wird gewährleistet, dass ein möglicher Kontakt der Oberfläche der zu behandelnden Lebensmittel mit der Oberfläche der Mittel zur Behandlung von Lebensmitteln zu keiner Beeinträchtigung der Qualität der Lebensmittel führen kann. Dies ist bei Fleisch und Fisch, aber auch bei der Behandlung von Käse und anderen Milchprodukten von Vorteil. Es ist dabei zu gewährleisten, dass die Wechselwirkung und das Zusammenspiel der Flüssigkeit bzw. Flüssigkeiten bewirkt, dass das Abtropfwasser Trinkwasserqualität aufweist

Die Elektrolyse der Reinigungsflüssigkeit wird bevorzugt als sogenannte Chloralkalielektrolyse durchgeführt. Eine Chloralkalielektrolyse wird vorzugsweise als Diaphragmaverfahren durchgeführt, bei dem eine poröse, stromdurchlässige Scheidewand (Diaphragma) den Anodenraum vom Kathodenraum abtrennt. Durch Durchführung lediglich einer schwachen Elektrolyse und dadurch, dass die Elektrolysezelle kontinuierlich betrieben wird, ist es möglich, dass nicht die Natronlauge- bzw. Chlorproduktion im Vordergrund steht, sondern die Bildung von partiellen Oxidationsprodukten im Anodenraum bzw. Reduktionsprodukten im Kathodenraum. Beim Durchlauf einer Natriumchloridlösung durch die Anodenkammer bilden sich demzufolge oxidierende Substanzen, wie Chlor (in geringen Mengen), Hypochlorit, Chlorit, Chlordioxid, Chlorat und andere Oxidationsmittel. Bei der Untersuchung des desinfizierenden Effekts der elektrochemisch behandelten Reinigungsflüssigkeit konnte gezeigt werden, dass selbst nach Entfernen eines Großteils bzw. aller oxidierenden Verbindungen aus einer mit Elektrodiaphragmalyse behandelten Natriumchloridlösung die desinfizierende Wirkung nicht beeinträchtigt wird.

Es wurde bisher angenommen, dass bei diesen Verfahren Natriumhypochlorit und andere Oxidantien für die desinfizierende Wirkung verantwortlich sind, wobei angenommen wurde, dass die Oxidantien bei Verwendung oxidierend auf die Umgebung reagieren und z.B. bakterielle Zellmembranen denaturieren.

Da diese oxidierenden Verbindungen in der Reinigungsflüssigkeit in einer sehr geringen Menge vorliegen oder sogar ganz entfernt wurden, muss in der Reinigungsflüssigkeit bzw. dem Wasser ein anderer Wirkmechanismus vorliegen. Es wird angenommen, dass die Wirkung des Wassers auf der Anregung des Wassermoleküls selbst beruht. Die Wassermoleküle befinden sich in einem Clusterverbund, so dass durch Durchführung einer schwachen Elektrolyse Wassermoleküle elektrisch entladen werden und die erzeugten Ladungsträger im Clusterverbund durch ständigen Austausch stabilisiert sind. Das elektrisch entladene Wasser kann daher dennoch desinfizierend wirken, da es imstande ist, Zellstrukturen zu denaturieren bzw. die Elektronentransport-Mechanismen von Mikroorganismen unwiderruflich zu stören. Dies ist einer der Gründe für die fehlende Resistenzbildungen von Mikroorganismen gegenüber dem mittels Elektrolyse behandelten Wasser.

Wegen der fehlenden Zerlegung des Wassers in der durchgeführten schwachen Elektrolyse kann das Wasser mit einem pH von 7 hergestellt werden. Dies ist insbesondere bei der Anwendung in pH-sensitiven Anwendungen, wie der Fischzucht und der Behandlung von Lebensmitteln, besonders bevorzugt.

Das durch Elektrolyse gewonnene Wasser hat eine umfassende Wirkung gegen Bakterien, Pilze, Viren, Prionen, Staphylococcus aureus, Bacillus pynocyaneus, Escherichia coli, Salmonellen, Bakterien-Sporen, Hepatitis-B Virus, Poliomyelitis Virus, HIV, Adenoviren, Hautpilze und Legionellen. Verschiedene Algenarten werden ebenfalls sicher abgetötet.

Es kann unter Umständen auch vorteilhaft sein, eine mittels Elektrolyse gewonnene Reinigungsflüssigkeit einzusetzen, die einen alkalischen pH-Wert aufweist. Ein alkalischer pH-Wert kann durch einen Gehalt an NaOH bewirkt werden. Neben anderen reduzierenden Spezies kann eine alkalische Reinigungsflüssigkeit als Produkte auch Nitrit, Chlorid, Phosphat, Sulfat, Nitrat, Natrium und Kalium in unterschiedlichen, aber sehr geringen Konzentrationen enthalten.

Dabei kann die saure bzw. die alkalische Reinigungsflüssigkeit je nach Bedarf eingesetzt werden: Es kann mitunter von Vorteil sein, eine Mischung beider Flüssigkeiten, anzuwenden - es kann auch von Vorteil sein, beide Flüssigkeiten nacheinander anzuwenden. Bei der Entfernung von Fett- und Eiweißresten beispielsweise ist bevorzugt die alkalische Flüssigkeit zuerst anzuwenden, da diese Fett- und Eiweiß-Reste wirkungsvoll entfernt, und dann die saure bis neutrale Flüssigkeit anzuwenden, die besonders wirkungsvoll Keime und Mikroorganismen abtötet.

Von besonderem Vorteil ist es dabei, wenn die Reinigung eine desinfizierende Wirkung auf Bakterien, Bakteriensporen, Pilzen, Pilzsporen, Viren, Prionen oder Mischungen davon aufweist. Eine Abtötung solcher Mikroorganismen bewirkt eine hohe hygienische Qualität bei der Lebensmittelverarbeitung.

Dabei ist es auch vorteilhaft, wenn in einer weiteren bevorzugten Ausführungsform die Reinigung einen Zeitraum zwischen 1 und 180 Sekunden, bevorzugt zwischen 5 und 120 Sekunden, und besonders bevorzugt zwischen 10 und 15 Sekunden benötigt. Insbesondere in Schlachtbetrieben werden Lebensmitteln in einer großen Menge verarbeitet. Die Desinfektion muss daher in einem Schlachtbetrieb sehr schnell erfolgen, da die Durchlaufgeschwindigkeit der zu verarbeitenden Tiere sehr hoch ist. Daher ist es von besonderem Vorteil wenn eine Reinigung zugleich wirksam und schnell erfolgt. Unter besonderen Voraussetzungen, so z.B. nach Abschluss der Arbeiten ist auch eine Reinigung möglich, die einen Zeitraum kleiner als 5 Minuten benötigt.

Es hat sich als vorteilhaft erwiesen, wenn in einer weiteren bevorzugten Ausführungsform das Mittel zur Beaufschlagung der Oberflächen des Mittels zur Behandlung von Lebensmitteln mit einer Reinigungsflüssigkeit eine Düse ist. Über eine Düse kann einerseits eine sehr zielgerichtete und fokussierte Beaufschlagung einer Oberfläche erzielt werden, andererseits können über die Anordnungen verschiedener Verteilungen von Düsen unterschiedliche Reinigungsmuster erzielt werden.

Von besonderem Vorteil ist es dabei, wenn die Düse eine Flachstrahldüse und/oder eine Rundstrahldüse und/oder eine Vollkegeldüse ist. Je nach Art und Beschaffenheit des betreffenden Mittels zur Behandlung von Lebensmitteln kann die entsprechende Düse ausgewählt bzw. angepasst werden.

Von besonderem Vorteil ist es dabei, wenn in einer weiteren bevorzugten Ausführungsform die Düse eine pulsierende Düse und/oder eine rotierende Düse ist. Über eine pulsierende Düse kann erreicht werden, dass mit verhältnismäßig geringem Flüssigkeitsaufwand bevorzugt eine hohe Strahlintensität erzielt wird; über eine rotierende Düse kann erreicht werden, dass bei verhältnismäßig geringen Flüssigkeitsaufwand bevorzugt eine relativ große Oberfläche mit Reinigungsflüssigkeit beaufschlagt werden kann.

Dabei ist es von besonderem Vorteil, wenn in einer weiteren bevorzugten Ausführungsform die Beaufschlagung des Mittels zur Behandlung von Lebensmittel dauernd und/oder intermittierend ausführbar ist. So kann eine dauernde Beaufschlagung vorteilhaft sein, wenn das Mittel zur Behandlung von Lebensmittel intensiv genutzt wird und eine schnelle Reinigung gewünscht ist. Eine intermittierende Beaufschlagung hingegen kann vorteilhaft sein, wenn mehr Zeit zur Verfügung steht und verstärkt energiesparende Reinigungsmethoden gewünscht sind.

Weiterhin ist es gemäß der Erfindung von Vorteil, dass die Reinigungsflüssigkeit eine Temperatur zwischen 5°C und 60°C aufweist. Bei Temperaturen über 60°C neigen Einweiß- und Fettreste dazu zu koagulieren, so dass sich Rückstände auf den Mitteln zur Behandlung der Lebensmittel bilden und eine erfolgreiche Desinfektion bzw. Sterilisation deswegen nicht mehr zuverlässig durchgeführt werden kann. Bakterien, die durch Fett- oder Eiweißrückstände umschlossen sind, können durch Desinfektionsmaßnahmen nicht erreicht werden. Wird eine Temperatur zwischen 5°C und 60°C gewählt, so kann auf der einen Seite eine solche mögliche Koagulation vermieden werden und gleichzeitig ein möglichst hoher Desinfektionserfolg gewährleistet werden. Es kann auch von Vorteil sein, als Reinigungsflüssigkeit eine Mischung der alkalischen sowie der sauren bis neutralen Reinigungsflüssigkeiten bei einer Temperatur zwischen 5°C und 60°C anzuwenden; es kann auch von Vorteil sein, eine der beiden Flüssigkeiten bei einer Temperatur zwischen 5°C und 60°C anzuwenden und die andere bei Raumtemperatur, dabei ist bevorzugt die alkalische Reinigungsflüssigkeit bei einer Temperatur zwischen 5°C und 60°C anzuwenden, da diese Reinigungsflüssigkeit aufgrund ihrer alkalischen Eigenschaften vorteilhafte Eigenschaften bei der Entfernung von Fettresten aufweist, welche durch die Temperatur zwischen 5°C und 60°C noch verbessert werden. Es können aber auch beide Reinigungsflüssigkeiten bei Raumtemperatur oder anderen Temperaturen eingesetzt werden, was besonders energiesparend ist.

Es hat sich als vorteilhaft erwiesen, wenn in einer bevorzugten Ausführungsform die Vorrichtung zur Reinigung eines Mittels zur Behandlung von Lebensmitteln einer Halterung eines Mittels zur Behandlung von Lebensmitteln aufweist. Dies ermöglicht es, das Mittel zur Behandlung von Lebensmitteln zur Reinigung in die Vorrichtung einzubringen und sie dort zu belassen. Dies ermöglicht es dem Anwender, während des Reinigungsvorganges sich anderen Tätigkeiten zu zuwenden.

Von besonderem Vorteil ist es dabei, wenn in einer weiteren bevorzugten Ausführungsform bei Einstecken des Mittels zur Behandlung von Lebensmitteln in die Halterung eine Beaufschlagung mit Reinigungsflüssigkeit automatisch auslösbar ist. Dies bewirkt einerseits eine weitere Entlastung des Anwenders sowie andererseits in verstärktem Maße eine zuverlässige Desinfektion des Mittels zur Behandlung von Lebensmitteln.

Weiterhin ist es von Vorteil, wenn in einer bevorzugten Ausführungsform über das Mittel zur Beaufschlagung der Oberfläche des Mittels zur Behandlung von Lebensmitteln mit einer Reinigungsflüssigkeit der Reinigungsflüssigkeit Wasser in einer Konzentration von 0,1% bis 100% beimischbar ist. Über die Variation des Gehaltes von Wasser kann einerseits die Intensität der Desinfektion in Abhängigkeit von dem zu behandelnden Lebensmittel bzw. in Abhängigkeit von der Anzahl der Vorgänge gesteuert werden. Anderseits kann über eine Variation der Konzentration eine Anpassung des Energieverbrauchs erfolgen.

Es kann auch von Vorteil sein, wenn in einer bevorzugten Ausführungsform als Reinigungsflüssigkeit eine Mischung beider Reinigungsflüssigkeiten angewendet wird; dabei können beide Zusätze einen Anteil zwischen 0,1% und 100% aufweisen. Es kann in Abhängigkeit von den technischen Voraussetzungen aber auch von Vorteil sein, beide Flüssigkeiten getrennt oder nacheinander anzuwenden bzw. nur einen von beiden; auch hier ist ein Anteil der betreffenden Reinigungsflüssigkeit zwischen 0,1% und 100% möglich. Werden beide Reinigungsflüssigkeiten nacheinander angewendet, so ist es von Vorteil, wenn zuerst die alkalische Reinigungsflüssigkeit eingesetzt wird, um Fett- und Eiweißreste abzulösen. In einem zweiten Schritt kann dann die saure bis neutrale Reinigungsflüssigkeit eingesetzt werden, um keimtötend zu wirken.

Ein zusätzlicher Reinigungseffekt kann in einer weiteren bevorzugten Ausführungsform durch den zusätzlichen Einsatz von Druckluft und/oder Heißluft und/oder UV-Licht und/oder einem Tensid erfolgen.

Darüber hinaus kann die Vorrichtung in einer weiteren bevorzugten Ausführungsform zusätzliche mechanische Mittel wie Bürsten und/oder Schwämme und/oder Reinigungskörper aufweisen, welche die Reinigung zusätzlich unterstützen. Gelöst wird die Aufgabe, ein verbessertes Verfahren zur Reinigung eines Mittels zum Behandeln von Lebensmitteln in einem Schlachtbetrieb zur Verfügung zu stellen, durch ein Verfahren, welches die Schritte aufweist:
a. Behandeln einer Reinigungsflüssigkeit (6) mittels Elektrolyse,
b. Einbringen des Mittels (2) zum Behandeln von Lebensmitteln in die Vorrichtung (1),
c. Beaufschlagung der Oberflächen (5a, 5b) des Mittels (2) zur Behandlung von Lebensmitteln mit der Reinigungsflüssigkeit (6).

Dieses Verfahren ermöglicht eine schnelle und effektive Reinigung der Mittel zum Behandeln von Lebensmitteln.

In einer bevorzugten Ausführungsform wird der Reinigungsflüssigkeit vor der Elektrolyse NaCl zugesetzt.

Dabei ist es von besonderem Vorteil, wenn in einer bevorzugten Ausführungsform die Reinigungsflüssigkeit Wasser ist.

Als vorteilhaft hat es sich auch erwiesen, wenn in einer bevorzugten Ausführungsform die Reinigung einen Zeitraum zwischen 1 und 180 Sekunden, bevorzugt zwischen 5 und 120 Sekunden, und besonders bevorzugt zwischen 10 und 15 Sekunden benötigt. Bei der Fleischverarbeitung ist aus hygienischen und ökonomischen Gründen eine schnelle Reinigung der Messer unerlässlich.

In einer bevorzugten Ausführungsform erfolgt die Beaufschlagung der Oberflächen des Mittels zur Behandlung von Lebensmitteln mit einer Reinigungsflüssigkeit mit wenigstens einer Düse.

Von besonderem Vorteil ist es dabei wenn die Reinigung des Mittels zur Behandlung von Lebensmitteln mit einer Reinigungsflüssigkeit mit einer Flachstrahldüse und/oder einer Rundstrahldüse und/oder einer Vollkegeldüse erfolgt.

Von ganz besonderem Vorteil ist dabei, wenn in bevorzugter Weise die Beaufschlagung der Oberflächen des Mittels zur Behandlung von Lebensmitteln mit einer Reinigungsflüssigkeit mit einer pulsierenden Düse und/oder eine rotierenden Düse erfolgt.

Als vorteilhaft hat es sich auch erwiesen, wenn in einer weiteren bevorzugten Ausführungsform die Beaufschlagung des Mittels zur Behandlung von Lebensmitteln dauernd und/oder intermittierend erfolgt.

In einer weiteren bevorzugten Ausführungsform weist die Vorrichtung wenigstens ein Mittel zur Erwärmung der Reinigungsflüssigkeit auf. Von ganz besonderem Vorteil ist es dabei, wenn die Reinigungsflüssigkeit über das Mittel zur Erwärmung der Reinigungsflüssigkeit auf eine Temperatur zwischen 5°C und 60°C erwärmt wird. Bei dieser Temperatur wird eine Reinigung und Desinfektion verstärkt, ohne dass eine Koagulation von Eiweiß- bzw. Fettresten zu befürchten wäre.

Es hat sich auch als vorteilhaft erwiesen, wenn das Mittel zur Behandlung von Lebensmitteln durch eine Halterung gehaltert wird. Dies ermöglicht es, das Mittel zur Behandlung von Lebensmitteln während der Reinigung dort zu belassen. Von ganz besonderem Vorteil ist es dabei, wenn beim Einstecken des Mittels zur Behandlung von Lebensmitteln in die Halterung eine Beaufschlagung der Oberflächen des Mittels mit Reinigungsflüssigkeit automatisch ausgelöst wird.

In einer weiteren bevorzugten Ausführungsform wird der Reinigungsflüssigkeit Wasser in einer Konzentration von 0,1% bis 100% beigemischt.

Ein zusätzlicher Nutzen kann beobachtet werden, wenn dass das Mittel zur Behandlung von Lebensmitteln zusätzlich mit Druckluft und/oder Heißluft und/oder UV-Licht und/oder einem Tensid beaufschlagt wird. Dies ermöglicht eine besonders intensive Reinigung.

Dabei ist es von besonderem Vorteil, wenn in einer weiteren bevorzugten Ausführungsform das Mittel zur Behandlung von Lebensmitteln durch zusätzliche Mittel wie Bürsten und/oder Schwämme und/oder Reinigungskörper mechanisch gereinigt wird.

Als vorteilhaft hat sich die Verwendung des Verfahrens zur Reinigung von Messern in einem Schlachthof erwiesen.

Weitere Vorteile und Ausgestaltungen der Erfindung werden im Folgenden anhand von zwei Ausführungsbeispielen veranschaulicht.

Darin zeigen schematisch:
Fig. 1 ein erstes Ausführungsbeispiel einer Vorrichtung zur Reinigung eines Mittels zur Behandlung von Lebensmitteln; sowie
Fig. 2 eine zweites Ausführungsbeispiel einer Vorrichtung zur Reinigung eines Mittels zur Behandlung von Lebensmitteln.

Bei der nachfolgenden Beschreibung zweier Ausführungsformen einer Vorrichtung bezeichnen gleiche Bezugszeichen gleiche oder vergleichbare Komponenten.

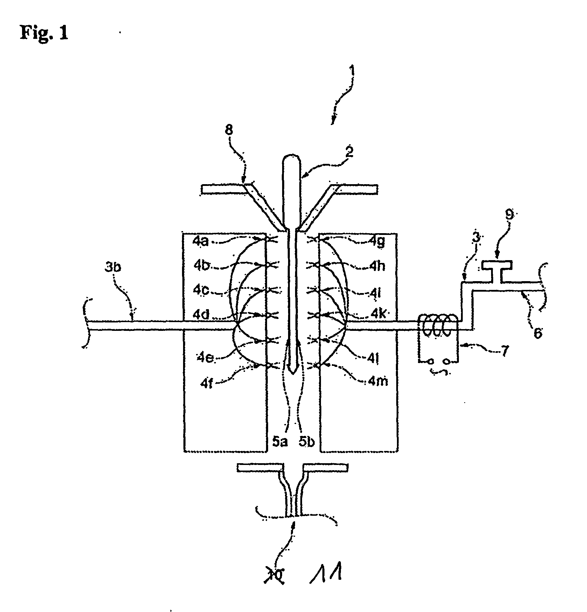

Fig. 1 zeigt in einer seitlichen Querschnittszeichnung eine Vorrichtung 1 zur Reinigung eines Mittels 2 zur Behandlung von Lebensmitteln. Das Mittel 2 zur Behandlung von Lebensmitteln ist in dieser Ausführungsform ein Messer. Der Griff des Messers 2 wird durch eine Halterung 8 gehalten. Über einen Flüssigkeitszulauf 3 strömt Reinigungsflüssigkeit 6 in die Vorrichtung 1 zur Reinigung eines Mittels 2 zur Behandlung von Lebensmitteln. Über ein Ventil 9 ist der Zufluss der Reinigungsflüssigkeit 6 steuer- bzw. regelbar. Weiterhin weist die Vorrichtung 1 ein Mittel 7 zur Erwärmung der Reinigungsflüssigkeit 6 auf. Dies Mittel 7 ist in der gezeigten Ausführungsform eine Heizspirale. Die erwärmte Reinigungsflüssigkeit 6 gelangt in das Innere der Vorrichtung 1 zu den Mitteln 4a bis 4m zur Beaufschlagung der Oberflächen 5a und 5b. Diese Mittel 4a bis 4m sind in dieser Ausführungsform Düsen. Über die Düsen 4a bis 4m werden die Oberflächen 5a und 5b des Mittels 2 zur Behandlung von Lebensmitteln mit der Reinigungsflüssigkeit 6 beaufschlagt. Über einen Ablauf 11 verlässt die Reinigungsflüssigkeit 6 nach erfolgter Reinigung anschließend wieder die Vorrichtung 1.

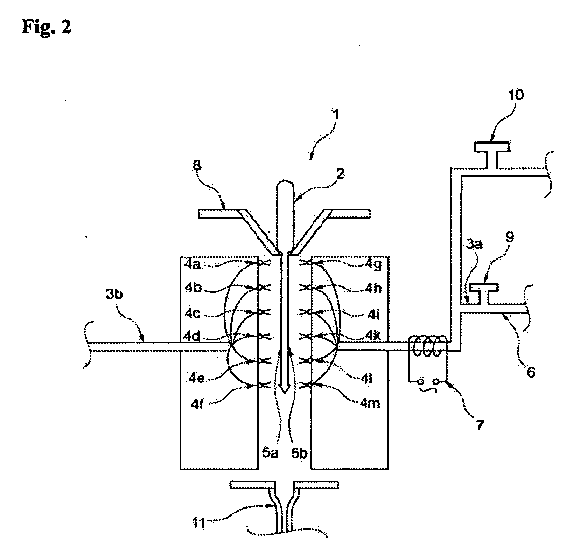

Fig. 2 zeigt eine zweite Ausführungsform der Vorrichtung 1 zur Reinigung eines Mittels 2 zur Behandlung von Lebensmitteln. Über einen Flüssigkeitszulauf 3 gelangt eine Reinigungsflüssigkeit 6 in das Innere der Vorrichtung 1, von wo es zu den Mitteln 4a bis 4m zur Beaufschlagung der Oberflächen 5a und 5b des Mittels 2 zur Behandlung von Lebensmitteln gelangt. Die Mittel 4a bis 4m zur Beaufschlagung der Oberflächen 5a und 5b sind in dieser Ausführungsform Düsen. Über die Düsen 4a bis 4m wird die Reinigungsflüssigkeit 6 auf die Oberflächen 5a und 5b des Mittels 2 zur Behandlung von Lebensmitteln gesprüht. Das Mittel 2 zur Behandlung von Lebensmitteln ist in dieser Ausführungsform ein Messer. Das Messer 2 wird durch eine Halterung 8 gehalten. Über ein Mittel 7 zur Erwärmung der Reinigungsflüssigkeit 6 wird die Reinigungsflüssigkeit 6 erwärmt. Dies Mittel 7 ist in der gezeigten Ausführungsform eine Heizspirale. Über ein Zusatzventil 10 kann je nach Bedarf zusätzliche Reinigungsflüssigkeit 6 oder Wasser zudosiert werden. Dies ermöglicht es, das Volumen bzw. die Konzentration der Reinigungsflüssigkeit 6 zu variieren. Über einen Abfluss 11 verlässt die Reinigungsflüssigkeit 6 anschließend wieder die Vorrichtung.

### Ausführungsformen einer Vorrichtung :

1. Ausführungsform : Vorrichtung (1) zur Reinigung eines Mittels (2) zur Behandlung von Lebensmitteln, insbesondere Messer, wobei die Vorrichtung Teil eines Schlacht- und/oder Zerlegebetriebes ist, die Vorrichtung wenigstens umfassend einen Flüssigkeitszulauf(3) sowie ein Mittel (4) zur Beaufschlagung der Oberflächen (5a, 5b) des Mittels (2) zur Behandlung von Lebensmitteln mit einer Reinigungsflüssigkeit (6), dadurch gekennzeichnet, dass über das Mittel (4) zur Beaufschlagung der Oberflächen (5a, 5b) des Mittels (2) zur Behandlung von Lebensmitteln mit einer Reinigungsflüssigkeit (6) eine mittels Elektrolyse behandelte Flüssigkeit einsprühbar ist.
2. Vorrichtung (1) zur Reinigung eines Mittels (2) zur Behandlung von Lebensmitteln nach Ausführungsform 1, dadurch gekennzeichnet, dass die Gesamtkonzentration der bei der Elektrolyse der Reinigungsflüssigkeit (6) entstandenen Oxidantien kleiner als 500 ppm, bevorzugt kleiner als 200 ppm, besonders bevorzugt kleiner als 20 ppm, ganz besonders bevorzugt kleiner als 2 ppm, und insbesondre besonders bevorzugt kleiner 0,2 ppm ist.
3. Vorrichtung (1) zur Reinigung eines Mittels (2) zur Behandlung von Lebensmitteln nach Ausführungsform 1 oder 2, dadurch gekennzeichnet, dass der Reinigungsflüssigkeit (6) vor der Elektrolyse NaCl zugesetzt wurde.
4. Vorrichtung (1) zur Reinigung eines Mittels (2) zur Behandlung von Lebensmitteln nach einem der vorhergehenden Ausführungsformen, dadurch gekennzeichnet, dass die Reinigungsflüssigkeit eine desinfizierende Wirkung auf Bakterien, Bakteriensporen, Pilzen, Pilzsporen, Viren, Prionen oder Mischungen davon aufweist.
5. Vorrichtung (1) zur Reinigung eines Mittels (2) zur Behandlung von Lebensmitteln nach einem der vorhergehenden Ausführungsformen, dadurch gekennzeichnet, dass die Reinigung einen Zeitraum zwischen 1 und 180 Sekunden, bevorzugt zwischen 5 und 120 Sekunden, und besonders bevorzugt zwischen 10 und 15 Sekunden benötigt
6. Vorrichtung (1) zur Reinigung eines Mittels (2) zur Behandlung von Lebensmitteln nach einem der vorhergehenden Ausführungsformen, dadurch gekennzeichnet, dass das Mittel (4) zur Beaufschlagung der Oberflächen (5a, 5b) des Mittels (2) zur Behandlung von Lebensmitteln mit einer Reinigungsflüssigkeit (6) eine Düse (4) ist.
7. Vorrichtung (1) zur Reinigung eines Mittels (2) zur Behandlung von Lebensmitteln nach Ausführungsform 6, dadurch gekennzeichnet, dass die Düse (4) eine Flachstrahldüse und/oder eine Rundstrahldüse und/oder eine Vollkegeldüse ist.
8. Vorrichtung (1) zur Reinigung eines Mittels (2) zur Behandlung von Lebensmitteln nach Ausführungsform 6 oder 7 dadurch gekennzeichnet, dass die Düse (4) eine pulsierende Düse und/oder eine rotierende Düse ist.
9. Vorrichtung (1) zur Reinigung eines Mittels (2) zur Behandlung von Lebensmitteln nach einem der vorhergehenden Ausführungsformen, dadurch gekennzeichnet, dass eine Beaufschlagung des Mittels (2) zur Behandlung von Lebensmitteln dauernd und/oder intermittierend ausführbar ist.
10. Vorrichtung (1) zur Reinigung eines Mittels (2) zur Behandlung von Lebensmitteln nach einem der vorhergehenden Ausführungsformen, dadurch gekennzeichnet, dass die Vorrichtung (1) wenigstens ein Mittel (7) zur Erwärmung der Reinigungsflüssigkeit (6) aufweist.
11. Vorrichtung (1) zur Reinigung eines Mittels (2) zur Behandlung von Lebensmitteln nach einem der vorhergehenden Ausführungsformen, dadurch gekennzeichnet, dass die Reinigungsflüssigkeit (6) eine Temperatur zwischen 5°C und 60°C aufweist.
12. Vorrichtung zur Reinigung eines Mittels zur Behandlung von Lebensmitteln nach einem der vorhergehenden Ausführungsformen, dadurch gekennzeichnet, dass die Vorrichtung (1) eine Halterung (8) für das Mittel (2) zur Behandlung von Lebensmitteln aufweist.
13. Vorrichtung (1) zur Reinigung eines Mittels (2) zur Behandlung von Lebensmitteln nach Ausführungsform 12, dadurch gekennzeichnet, dass beim Einstecken des Mittels (2) zur Behandlung von Lebensmitteln in die Halterung (8) eine Beaufschlagung der Oberflächen (5a, 5b) des Mittels (2) mit Reinigungsflüssigkeit (6) automatisch auslösbar ist.
14. Vorrichtung (1) zur Reinigung eines Mittels (2) zur Behandlung von Lebensmitteln nach einem der vorhergehenden Ausführungsformen, dadurch gekennzeichnet, dass über das Mittel (4) zur Beaufschlagung der Oberflächen (5a, 5b) des Mittels (2) zur Behandlung von Lebensmitteln mit einer Reinigungsflüssigkeit (6) der Reinigungsflüssigkeit (6) Wasser in einer Konzentration von 0,1% bis 100% beimischbar ist.
15. Vorrichtung (1) zur Reinigung eines Mittels (2) zur Behandlung von Lebensmitteln nach einem der vorhergehenden Ausführungsformen, dadurch gekennzeichnet, dass die Vorrichtung (1) zusätzliche Mittel zur Beaufschlagung des Mittels (2) zur Behandlung von Lebensmitteln mit einer Reinigungsflüssigkeit (6) mit Druckluft und/oder Heißluft und/oder UV-Licht und/oder einem Tensid aufweist.
16. Vorrichtung (1) zur Reinigung eines Mittels (2) zur Behandlung von Lebensmitteln nach einem der vorhergehenden Ausführungsformen, dadurch gekennzeichnet, dass die Vorrichtung (1) zusätzliche mechanische Mittel wie Bürsten und/oder Schwämme und/oder Reinigungskörper aufweist, welche die Reinigung zusätzlich mechanisch unterstützen.

Durch das erfindungsgemäße Verfahren wird eine kontinuierliche, energiesparende und zugleich zuverlässige Reinigung und Desinfektion eines Mittels zur Behandlung von Lebensmitteln gewährleistet.

### Bezugszeichenliste

- 1: Vorrichtung zur Reinigung
- 2: Mittel zur Behandlung von Lebensmitteln; Messer
- 3: Flüssigkeitszulauf
- 4a - 4m: Mittel zur Beaufschlagung der Oberfläche; Düse
- 5: Oberfläche des Mittels zur Behandlung von Lebensmitteln
- 6: Reinigungsflüssigkeit
- 7: Mittel zur Erwärmen der Reinigungsflüssigkeit
- 8: Halterung
- 9: Ventil
- 10: Zusatzventil
- 11: Abfluss

## Patentansprüche

1. Verfahren zur Reinigung eines Mittels (2) zum Behandeln von Lebensmitteln, insbesondere Messer, in einem Schlacht- und/oder Zerlegebetrieb mittels einer Vorrichtung (1) nach einem der vorhergehenden Patentansprüche, **gekennzeichnet durch** folgende Schritte:
a. Behandeln einer Reinigungsflüssigkeit (6) mittels Elektrolyse,
b. Einbringen des Mittels (2) zum Behandeln von Lebensmitteln in die Vorrichtung (1), wobei die Vorrichtung Teil eines Schlacht- und/oder Zerlegebetriebes ist, die Vorrichtung wenigstens umfassend einen Flüssigkeitszulauf (3) sowie ein Mittel (4) zur Beaufschlagung der Oberflächen (5a, 5b) des Mittels (2) zur Behandlung von Lebensmitteln mit einer Reinigungsflüssigkeit (6) umfasst und dass über das Mittel (4) zur Beaufschlagung der Oberflächen (5a, 5b) des Mittels (2) zur Behandlung von Lebensmitteln mit der mittels Elektrolyse behandetten Reinigungsflüssigkeit (6) einsprühbar ist.
c. Beaufschlagung der Oberflächen (5a, 5b) des Mittels (2) zum Behandeln von Lebensmitteln mit der Reinigungsflüssigkeit (6), wobei die Vorrichtung (1) wenigstens ein Mittel (7) zur Erwärmung der Reinigungsflüssigkeit (6) aufweist und die Reinigungsflüssigkeit (6) über das Mittel (7) auf eine Temperatur zwischen 5°C und 60°C erwärmt wird, wobei auf der einen Seite eine mögliche Koagulation von Eiweiß- und Fettresten vermieden wird, und gleichzeitig ein möglichst hoher Desinfektionserfolg gewährleistet werden kann.

2. Verfahren nach Patentanspruch 1, **dadurch gekennzeichnet, dass** der Reinigungsflüssigkeit (6) vor der Elektrolyse NaCl zugesetzt wird.

3. Verfahren nach Patentanspruch 2, **dadurch gekennzeichnet, dass** die Reinigungsflüssigkeit (6) Wasser ist.

4. Verfahren zur Reinigung eines Mittels (2) zur Behandlung von Lebensmitteln nach Patentanspruch 1 oder 23, **dadurch gekennzeichnet, dass** aus der Reinigungsflüssigkeit nach der Elektrolyse Oxidantien entfernt werden, so dass deren Gesamtkonzentration kleiner als 500 ppm, bevorzugt kleiner als 200 ppm, besonders bevorzugt kleiner als 20 ppm, ganz besonders bevorzugt kleiner als 2 ppm, und insbesondre besonders bevorzugt kleiner 0,2 ppm ist.

5. Verfahren zur Reinigung eines Mittels (2) zur Behandlung von Lebensmitteln nach Patentanspruch 1 bis 4, **dadurch gekennzeichnet, dass** die Reinigung einen Zeitraum zwischen 1 und 180 Sekunden, bevorzugt zwischen 5 und 120 Sekunden, und besonders bevorzugt zwischen 10 und 15 Sekunden benötigt.

6. Verfahren zur Reinigung eines Mittels (2) zur Behandlung von Lebensmitteln nach einem der Patentansprüche 1 bis 5, **dadurch gekennzeichnet, dass** die Beaufschlagung der Oberflächen (5a, 5b) des Mittels (2) zur Behandlung von Lebensmitteln mit einer Reinigungsflüssigkeit mit einer Düse (4) erfolgt.

7. Verfahren zur Reinigung eines Mittels (2) zur Behandlung von Lebensmitteln nach Patentanspruch 6, **dadurch gekennzeichnet, dass** die Beaufschlagung der Oberflächen (5a, 5b) des Mittels (2) zur Behandlung von Lebensmitteln mit einer Reinigungsflüssigkeit (6) mit einer Flachstrahldüse und/oder einer Rundstrahldüse und/oder einer Vollkegeldüse erfolgt.

8. Verfahren zur Reinigung eines Mittels (2) zur Behandlung von Lebensmitteln nach Patentanspruch 7, **dadurch gekennzeichnet, dass** die Beaufschlagung der Oberflächen (5a, 5b) des Mittels (2) zur Behandlung von Lebensmitteln mit einer Reinigungsflüssigkeit (6) mit einer pulsierenden Düse und/oder eine rotierenden Düse erfolgt.

9. Verfahren zur Reinigung eines Mittels (2) zur Behandlung von Lebensmitteln nach einem der Patentansprüche 1 bis 8, **dadurch gekennzeichnet, dass** eine Beaufschlagung des Mittels (2) zur Behandlung von Lebensmitteln dauernd und/oder intermittierend erfolgt.

10. Verfahren zur Reinigung eines Mittels (2) zur Behandlung von Lebensmitteln nach einem der Patentansprüche 1 bis 9, **dadurch gekennzeichnet, dass** das Mittel (2) zur Behandlung von Lebensmitteln durch eine Halterung (8) gehaltert wird.

11. Verfahren zur Reinigung eines Mittels (2) zur Behandlung von Lebensmitteln nach Patentanspruch 10, **dadurch gekennzeichnet, dass** beim Einstecken des Mittels (2) zur Behandlung von Lebensmitteln in die Halterung (8) eine Beaufschlagung der Oberflächen (5a, 5b) des Mittels (2) mit Reinigungsflüssigkeit (6) automatisch ausgelöst wird.

12. Verfahren zur Reinigung eines Mittels (2) zur Behandlung von Lebensmitteln nach einem der Patentansprüche 1 bis 11, **dadurch gekennzeichnet, dass** über das Mittel (4) zur Beaufschlagung der Oberflächen (5a, 5b) des Mittels (2) zur Behandlung von Lebensmitteln mit einer Reinigungsflüssigkeit (6) der Reinigungsflüssigkeit (6) Wasser in einer Konzentration von 0,1% bis 100% beigemischt wird.

13. Verfahren zur Reinigung eines Mittels (2) zur Behandlung von Lebensmitteln nach einem der Patentansprüche 1 bis 12, **dadurch gekennzeichnet, dass** das Mittel (2) zur Behandlung von Lebensmitteln zusätzlich mit Druckluft und/oder Heißluft und/oder UV-Licht und/oder einem Tensid beaufschlagt wird.

14. Verfahren zur Reinigung eines Mittels (2) zur Behandlung von Lebensmitteln nach einem der Patentansprüche 1 bis 13, **dadurch gekennzeichnet, dass** das Mittel (2) zur Behandlung von Lebensmitteln durch zusätzliche Mittel wie Bürsten und/oder Schwämme und/oder Reinigungskörper mechanisch gereinigt wird.

15. Verwendung eines Verfahrens nach einem der Patentansprüche 1 bis 14 zur Reinigung von Messern in einem Schlacht- und/oder Zerlegebetrieb.

## Claims

1. Process for cleaning a means (2) for the treatment of foodstuffs, in particular knifes, in a slaughter and/or meat-cutting company by means of an apparatus (1) according to one of the preceding claims **characterized by** the following steps:
a. Treating a cleansing fluid (6) by means of electrolysis,
b. Inserting the means (2) for the treatment of foodstuffs into the apparatus (1), wherein the apparatus is part of a slaughter or meat-cutting company, the apparatus at least comprising a fluid inlet (3) and as well comprises means (4) for applying a cleansing fluid (6) to the surfaces (5a, 5b) of the means (2) for the treatment of foodstuffs, and that via the means (4) for applying a cleansing fluid (6) to the surfaces (5a, 5b) of the means (2) for the treatment of foodstuffs, it is possible to inject a fluid (6) treated by means of electrolysis
c. Applying the cleansing fluid (6) to the surfaces (5a, 5b) of the means (2) for the treatment of foodstuffs,
whereby the apparatus (1) comprises at least one means (7) for heating the cleansing fluid (6) up and the cleansing fluid (6) is heated up to a temperature between 5°C and 60°C,
whereby on one hand a possible coagulation of residues of proteins and fats is avoided, and on the other hand a disinfection as successful as possible can be ensured.

2. Process according to patent claim 1, **characterized in that** before carrying out the electrolysis, NaCL is added to the cleansing fluid (6).

3. Process according to patent claim 2, **characterized in that** the cleansing fluid (6) is water.

4. Process for cleaning a means (2) for the treatment of foodstuffs according to patent claims 1 or 3, **characterized in that** after the electrolysis, oxidants are removed from the cleansing fluid such that their total concentration is less than 500 ppm, preferably less than 200 ppm, particularly preferably less than 20 ppm, very particularly preferably less than 2 ppm, and, in particular, particularly preferably less than 0.2 ppm.

5. Process for cleaning a means (2) for the treatment of foodstuffs according to patent claims 1 to 4, **characterized in that** the purification requires a time period between 1 and 180 seconds, preferably between 5 and 120 seconds, and particularly preferably between 10 and 15 seconds.

6. Process for cleaning a means (2) for the treatment of foodstuffs according to patent claims 1 to 5, **characterized in that** the application of a cleansing fluid to the surfaces (5a, 5b) of the means (2) for the treatment of foodstuffs is carried out by means of a nozzle (4).

7. Process for cleaning a means (2) for the treatment of foodstuffs according to patent claim 6, **characterized in that** the application of a cleansing fluid (6) to the surfaces (5a, 5b) of the means (2) for the treatment of foodstuffs is carried out by means of a flat jet nozzle and/or a round jet nozzle and/or a full-cone nozzle.

8. Process for cleaning a means (2) for the treatment of foodstuffs according to patent claim 7, **characterized in that** the application of a cleansing fluid (6) to the surfaces (5a, 5b) of the means (2) for the treatment of foodstuffs is carried out by means of a pulsating nozzle and/or a rotating nozzle.

9. Process for cleaning a means (2) for the treatment of foodstuffs according to one of the patent claims 1 to 8, **characterized in that** the application of the cleansing fluid to the means (2) for the treatment of foodstuffs is carried out permanently and/or intermittently.

10. Process for cleaning a means (2) for the treatment of foodstuffs according to one of the patent claims 1 to 9, **characterized in that** the means (2) for the treatment of foodstuffs is held by means of a holder (8).

11. Process for cleaning a means (2) for the treatment of foodstuffs according to patent claim 10, **characterized in that** when inserting the means (2) for the treatment of foodstuffs into the holder (8), the application of the cleansing fluid (6) to the surfaces (5a, 5b) of the means (2) is activated automatically.

12. Process for cleaning a means (2) for the treatment of foodstuffs according to one of the patent claims 1 to 11, **characterized in that** via the means (4) for applying a cleansing fluid (6) to the surfaces (5a, 5b) of the means (2) for the treatment of foodstuffs, water having a concentration of 0.1% to 100% is added to the cleansing fluid (6).

13. Process for cleaning a means (2) for the treatment of foodstuffs according to one of the patent claims 1 to 12, **characterized in that** pressurized air and/or hot air and/or ultraviolet light and/or a tenside is additionally applied to the means (2) for the treatment of foodstuffs.

14. Process for cleaning a means (2) for the treatment of foodstuffs according to one of the patent claims 1 to 13, **characterized in that** the means (2) for the treatment of foodstuffs is cleaned mechanically by additional means such as brushes, and/or sponges and/or cleaning bodies.

15. Use of a process according to one of the patent claims 1 to 14 for cleaning knifes in a slaughter and/or meat cutting company.

## Revendications

1. Procédé de nettoyage d'un moyen (2) pour traiter des produits alimentaires, en particulier de couteaux, dans un atelier d'abattage et/ou de découpe au moyen d'un dispositif (1) selon une des revendications précédentes, **caractérisé par** les étapes suivantes :
a. traitement d'un liquide de nettoyage (6) par électrolyse,
b. introduction du moyen (2) pour traiter des produits alimentaires dans le dispositif (1), le dispositif faisant partie d'un atelier d'abattage et/ou de découpe et comprenant au moins une arrivée de liquide (3) ainsi qu'un moyen (4) pour exposer les surfaces (5a, 5b) du moyen (2) pour traiter des produits alimentaires à un liquide de nettoyage (6), et un liquide de nettoyage (6) traité par électrolyse pouvant être projeté par le moyen (4) pour exposer les surfaces (5a, 5b) du moyen (2) pour traiter des produits alimentaires à un liquide de nettoyage (6),
c. exposition des surfaces (5a, 5b) du moyen (2) pour traiter des produits alimentaires à un liquide de nettoyage (6), le dispositif (1) présentant au moins un moyen (7) pour chauffer le liquide de nettoyage (6) et le liquide de nettoyage (6) étant chauffé par le moyen (7) à une température comprise entre 5 °C et 60 °C, une possible coagulation des restes de protéines et de graisse étant évitée et un résultat de désinfection maximal possible pouvant être garanti en même temps.

2. Procédé selon la revendication 1, **caractérisé en ce que** du NaCl est ajouté au liquide de nettoyage (6) avant l'électrolyse.

3. Procédé selon la revendication 2, **caractérisé en ce que** le liquide de nettoyage (6) est de l'eau.

4. Procédé de nettoyage d'un moyen (2) pour traiter des produits alimentaires selon la revendication 1 ou 3 , **caractérisé en ce que** des oxydants sont éliminés du liquide de nettoyage après l'électrolyse, de façon que leur concentration totale soit inférieure à 500 ppm, de préférence inférieure à 200 ppm, particulièrement de préférence inférieure à 20 ppm, tout particulièrement de préférence inférieure à 2 ppm et en particulier particulièrement de préférence inférieure à 0,2 ppm.

5. Procédé de nettoyage d'un moyen (2) pour traiter des produits alimentaires selon les revendications 1 à 4, **caractérisé en ce que** le nettoyage prend un temps compris entre 1 et 180 secondes, de préférence entre 5 et 120 secondes et particulièrement de préférence entre 10 et 15 secondes.

6. Procédé de nettoyage d'un moyen (2) pour traiter des produits alimentaires selon une des revendications 1 à 5, **caractérisé en ce que** l'exposition des surfaces (5a, 5b) du moyen (2) pour traiter des produits alimentaires à un liquide de nettoyage est réalisée avec une buse (4).

7. Procédé de nettoyage d'un moyen (2) pour traiter des produits alimentaires selon la revendication 6, **caractérisé en ce que** l'exposition des surfaces (5a, 5b) du moyen (2) pour traiter des produits alimentaires à un liquide de nettoyage (6) est réalisée avec une buse à jet plat et/ou une buse à jet rond et/ou une buse à cône plein.

8. Procédé de nettoyage d'un moyen (2) pour traiter des produits alimentaires selon la revendication 7, **caractérisé en ce que** l'exposition des surfaces (5a, 5b) du moyen (2) pour traiter des produits alimentaires à un liquide de nettoyage (6) est réalisée avec une buse pulsée et/ou une buse rotative.

9. Procédé de nettoyage d'un moyen (2) pour traiter des produits alimentaires selon une des revendications 1 à 8, **caractérisé en ce qu'**une application du moyen (2) pour traiter des produits alimentaires a lieu en continu et/ou par intermittence.

10. Procédé de nettoyage d'un moyen (2) pour traiter des produits alimentaires selon une des revendications 1 à 9, **caractérisé en ce que** le moyen (2) pour traiter des produits alimentaires est maintenu par un dispositif de maintien (8).

11. Procédé de nettoyage d'un moyen (2) pour traiter des produits alimentaires selon la revendication 10, **caractérisé en ce qu'**une exposition des surfaces (5a, 5b) du moyen (2) au liquide de nettoyage (6) est déclenchée automatiquement lors de l'insertion du moyen (2) pour traiter des produits alimentaires dans le dispositif de maintien (8).

12. Procédé de nettoyage d'un moyen (2) pour traiter des produits alimentaires selon une des revendications 1 à 11, **caractérisé en ce que** de l'eau est mélangée au liquide de nettoyage (6) dans une concentration de 0,1 % à 100 % par le moyen (4) pour exposer à un liquide de nettoyage (6) les surfaces (5a, 5b) du moyen (2) pour traiter des produits alimentaires.

13. Procédé de nettoyage d'un moyen (2) pour traiter des produits alimentaires selon une des revendications 1 à 12, **caractérisé en ce que** le moyen (2) pour traiter des produits alimentaires est exposé en plus à de l'air comprimé et/ou de l'air chaud et/ou une lumière UV et/ou un agent tensio-actif.

14. Procédé de nettoyage d'un moyen (2) pour traiter des produits alimentaires selon une des revendications 1 à 13, **caractérisé en ce que** le moyen (2) pour traiter des produits alimentaires est nettoyé mécaniquement par des moyens supplémentaires tels que des brosses et/ou des éponges et/ou des corps de nettoyage.

15. Utilisation d'un procédé selon une des revendications 1 à 14 pour nettoyer des couteaux dans un atelier d'abattage et/ou de découpe.
